Europäisches Patentamt

European Patent Office

Office européen des brevets

(11) Publication number: **0 404 003**
**A2**

(12) **EUROPEAN PATENT APPLICATION**

(21) Application number: **90111426.4**

(22) Date of filing: **18.06.90**

(51) Int. Cl.5: **C12N 15/13, C12N 1/21,**
**C12N 1/19, C12N 5/10,**
**C07K 15/28, C12P 21/02,**
**G01N 33/574**

The applicant has filed a statement in accordance with Rule 28 (4) EPC (issue of a sample only to an expert). Accession number(s) of the deposit(s): ATCC No. HB10131 and ATCC No. 20945.

(30) Priority: **19.06.89 US 367641**

(43) Date of publication of application:
**27.12.90 Bulletin 90/52**

(84) Designated Contracting States:
**AT BE CH DE DK ES FR GB GR IT LI LU NL SE**

(71) Applicant: **Xoma Corporation**
**2910 Seventh Street**
**Berkeley California 94710(US)**

(72) Inventor: **Better, Marc D.**
**2462 Zorada Lane**
**Los Angeles, California 90046(US)**
Inventor: **Horwitz, Arnold D.**
**7529 Midfield Avenue**
**Los Angeles, California 90045(US)**
Inventor: **Lei, Shau-Ping**
**11452 Clarkson Road**
**Los Angeles, California 90064(US)**
Inventor: **Robinson, Randy R.**
**7719 Isis Avenue**
**Los Angeles, California 90045(US)**

(74) Representative: **Kraus, Walter, Dr. et al**
**Patentanwälte Kraus, Weisert & Partner**
**Thomas-Wimmer-Ring 15**
**D-8000 München 22(DE)**

(54) Chimeric mouse-human KM10 antibody with specificity to a human tumor cell antigen.

(57) A chimeric antibody with human constant region and murine variable region, having specificity to human tumor cells, methods of production, and uses.

EP 0 404 003 A2

# CHIMERIC MOUSE-HUMAN KM10 ANTIBODY WITH SPECIFICITY TO A HUMAN TUMOR CELL ANTIGEN

## BACKGROUND OF THE INVENTION

### Field of the Invention

This invention relates to an antibody with human tumor cell specificity and its derivatives, nucleotide and protein sequences coding therefor, as well as methods of obtaining and manipulating such sequences.

### Background Art

Monoclonal antibody technology has greatly impacted current thinking about cancer therapy and diagnosis. The elegant application of cell-to-cell fusion for the production of monoclonal antibodies (mAb) by Kohler and Milstein (Nature 256:495 (1975)) spawned a revolution in biology equal in impact to that of recombinant DNA cloning. MAb produced from hybridomas are already widely used in diagnostic and basic scientific studies. Their efficacy in the treatment of human diseases including cancer, viral and microbial infections, to a large extent remains to be demonstrated.

Although mAb display exquisite specificity to targets that can influence the progression of human disease, mouse mAbs, by their very nature, have limitations in their applicability to human disease management. Most obviously, they are derived from mouse cells and when introduced into humans are recognized as foreign protein toward which an immune response may be elicited. Similarly, since they are distinguished from human proteins, they may be cleared at different rates from human circulation. Finally, since they are of mouse origin, they may not be recognized by the effector cells or human serum components as effectively as the human equivalent.

Technology to develop human mAb that could circumvent these particular problems has met with a number of obstacles. This is especially true when attempting to develop mAb which define human tumor antigens for the diagnosis and treatment of cancer. Many of these tumor antigens are not recognized as foreign antigens by the human immune system; therefore, these antigens may not be immunogenic in man. By contrast, those human tumor antigens that are immunogenic in mice can be used for the production of mouse mAb which specifically recognize the human antigen and may, therefore, have therapeutic utility in humans. In addition, many human mAb obtained in cell culture are of the IgM type. When it is desirable to obtain human monoclonals of the IgG type, it has been necessary to use such techniques as cell sorting, to identify and isolate the few cells which are producing antibodies of the IgG type. A need therefore exists for an efficient method of switching antibody classes, for any given antibody of a predetermined or desired antigenic specificity.

Chimeric antibodies, such as the one described in this invention, bridge both hybridoma and genetic engineering technologies and represent novel matter with potential in treatment and diagnosis of human cancer.

The chimeric antibody molecules of the present invention and their derivatives embody a combination of the advantageous characteristics of conventional mAb. The chimeric mAb, like mouse mAb, can recognize and bind to a human tumor antigen present in cancer tissue; however, unlike mouse mAb, the species-specificity of the chimeric antibodies will lower the likelihood of the induction of immune responses and will delay clearance when used in humans in vivo. The human-derived component of a chimeric antibody also may exhibit an enhanced ability to mediate target cell destruction in combination with effector cells and/or complement components of the human immune system. Moreover, using the methods disclosed in the present invention, any desired antibody isotype can be conferred upon a particular antigen combining site. The invention also enables the direct production of one or more domains of the antibody molecule in a functionally active form.

*Note: The present Information Disclosure Statement is subject to the provisions of 37 C.F.R. 1.97(b). In addition, Applicants reserve the right to demonstrate that their invention was made prior to any one or more of the mentioned publications.

INFORMATION DISCLOSURE STATEMENT *

Approaches to the problem of producing chimeric anti-tumor antibodies have been published by various authors.

Document AR1 (Horwitz, A.H. et al., Proc. Natl. Acad. Sci. U.S.A. 85:8676-8682 (1988)) demonstrates that whole chimeric antibodies and chimeric antibody fragments can be produced in the yeast Saccharomyces cerevisiae. Simultaneous expression of the antibody genes in yeast resulted in secretion of a properly folded and assembled chimeric antibody that bound to target cancer cells.

Document AS1 (Better, M. et al., Science 240:1041-1043 (1988)) describes the production of a chimeric mouse-human anti-carcinoma L6 Fab from E. coli. The genetically engineered Fab has the same binding properties as chimeric Fab prepared by papain digestion of chimeric L6 antibody produced in animal cells.

Document AL1 (Robinson, R.R. et al., International Patent Publication #PCT/US86/02269 (published 7 May 1987)) describes modular assembly of antibody genes, one example being the production of a chimeric L6 anti-carcinoma antibody.

Document AT1 (Liu, A.Y. et al., Proc. Natl. Acad. Sci.. USA 84:3439-3443 (1987)), describes the production of a mouse-human chimeric L6-antibody molecule that recognizes an antigen on the surface of many human carcinoma cells. Immunoglobulin constant domains of the mouse antibody were substituted by human constant domains using cDNA technology. Chimeric antibody produced from lymphoid cells and the parental mouse antibody bound to carcinoma cells with equal affinity and the chimeric antibody mediated complement-dependent cytolysis as well as antibody-dependent cellular cytotoxicity (ADCC).

Document AR2 (Liu, A.Y., et al., J. Immunol. 139:3521-3526 (1987)), describes a chimeric 2H7 mouse-human antibody molecule constructed using cDNA technology against a surface phosphoprotein on human B cells. The chimeric antibody exhibits properties not inherent in the parental mouse mAb, such as the ability to mediate ADCC and complement-dependent cytotoxicity.

Document AS2 (Beidler, C.B. et al., J. Immunol. 141:4053-4060 (1988)), describes the expression of a mouse-human chimeric antibody with specificity to the human carcinoma embryonic antigen (CEA). The document discloses high level expression of chimeric antibody from antibody genes constructed with genomic DNA fragments.

Document AT2 (Shaw, D.R., et al., J. Biol. Resp. Modifiers 7:204-211 (1988)), describes a chimeric antibody with the same antigen specificity as the mouse mAb 17-1A, recognizing an antigen expressed in human gastrointestinal malignancies. This chimeric antibody, expressed from genomic DNA fragments mediates ADCC.

Document AR3 (Sun, L.K., et al. Proc. Natl. Acad. Sci. USA 84:214-218 (1987)), discloses a chimeric antibody that binds to a surface antigen expressed on colorectal carcinoma cells. This antibody was constructed from genomic DNA fragments encoding the mouse variable region and human constant region, and the antibody was expressed in mouse myeloma cells.

Document AS3 (Nishimura, Y. et al., Canc. Res. 47:999-1005 (1987)), describes a mouse-human chimeric antibody directed against the common acute lymphocytic leukemia antigen (CALLA). This antibody was constructed from genomic DNA sequences encoding antibody variable domains from a mouse mAb and genomic DNA sequences encoding human antibody constant domains. The antibody bound specifically to cells expressing CALLA and could mediate both ADCC and complement- dependent cytolysis.

Document AM1 (Akira K. et al., European Patent Application 184187 (published June 11, 1986)) describes the mouse-human chimeric antibody directed against CALLA.

Document AT3 (Sahagan, B.G. et al., J. Immunol. 137:1066-1074 (1986)) discloses construction of a chimeric mouse-human antibody from genomic mouse variable and human constant DNA fragments. This antibody binds specifically to certain human carcinoma cell lines. The biodistribution of the chimeric antibody and the parental mouse mAb after injection into mice bearing tumors was identical.

Document AR4 (Brown, B.A. et al., Canc. Res. 47:3577-3583 (1987)) also discloses construction of a chimeric mouse-human antibody from genomic mouse variable and human constant DNA fragments. This antibody binds specifically to certain human carcinoma cell lines. The biodistribution of the chimeric antibody and the parental mouse mAb after injection into mice bearing tumors was identical.

Approaches to the general field of chimeric molecules, which may be applicable to the development of chimeric anti-tumor antibodies, have been disclosed by various authors.

Document AN1 (Taniguchi, M. European Patent Publication No. 171 496 (published February 19, 1985)), discloses the production of chimeric antibodies having variable regions with tumor specificity derived from experimental animals, and constant regions derived from the human. The corresponding heavy and light chain genes are derived from genomic DNA, and expressed in mammalian cells.

Document AS4 (Sun, L.K., et al., Hybridoma 5 (suppl. 1):S17 (1986)), describes a chimeric human/mouse antibody with potential tumor specificity. The chimeric heavy and light chain genes were genomic constructs and expressed in mammalian cells.

Document AT4 (Morrison, S.L. et al., Proc. Natl. Acad. Sci. USA 81:6851-6855 (1984)), describes the production of a mouse-human antibody molecule of defined antigen binding specificity, produced by joining the variable region genes of a mouse antibody-producing myeloma cell line with known antigen binding specificity to human immunoglobulin constant region genes using recombinant DNA techniques. Chimeric genes were constructed, wherein the heavy chain variable region exon from the myeloma cell line S107 was joined to human IgG1 or IgG2 heavy chain constant region exons, and the light chain variable region exon from the same myeloma was joined to the human x light chain exon. These genes were transfected into mouse myeloma cell lines, which then produced chimeric mouse-human antiphosphocholine antibodies.

Document AO1 (Morrison, S.L., et al., European Patent Publication No. 173494 (published March 5, 1986)), discloses chimeric "receptors" (e.g. antibodies) having variable regions derived from one species and constant regions derived from another. Mention is made of the possibility of utilizing cDNA cloning to obtain the genes, although no examples or teachings relating to cDNA priming and cloning or of the development of chimeric antibody genes thereby are shown (see pp. 5, 7 and 8).

Document AR5 (Boulianne, G.L. et al., Nature 312:643-646 (1984)), also disclosed the production of antibodies consisting of mouse variable regions joined to human constant regions. The document discloses construction of immunoglobulin genes in which the DNA segments encoding mouse variable regions specific for the hapten trinitrophenyl (TNP) were joined to segments encoding human $\mu$ and x constant regions. These chimeric genes were expressed as functional TNP-binding chimeric IgM antibodies in mammalian cells.

Document AS5 (Neuberger, M.S. et al. Nature 314:268 (1985)), discloses construction of a chimeric heavy chain immunoglobulin gene in which a DNA segment encoding a mouse variable region specific for the hapten 4-hydroxy-3-nitrophenacetyl (NP) was joined to segment encoding the human epsilon region. When an antibody coded for by this chimeric gene was produced, it bound to the NP hapten and had human IgE properties.

Document AT5, (Neuberger, M.S. et al., (Nature, 312:604-608 (1984)) discloses the preparation of cell lines that secrete hapten-specific antibodies in which the Fc portion has been replaced with a polypeptide displaying c-myc antigenic determinants.

Document AR6 (Williams, G. et al. (Gene 43:319-324 (1986)) discloses the preparation of cell lines that secrete hapten-specific antibodies in which the Fc portion has been replaced with an active enzyme moiety.

Document AP1 (Neuberger, M.S. et al., PCT Publication WO 86/01533, (published March 13, 1986)) discloses production of chimeric antibodies and suggests the concept of "class switching" as one of many conceptual uses of the general technology.

Document AS6 (Takeda, S. et al., Nature 314:452-454 (1985)), describes a potential method for the construction of chimeric immunoglobulin genes which have intron sequences removed by the use of a retrovirus vector. However, an unexpected splice donor site caused the deletion of the V region leader sequence. Thus, this approach did not yield complete chimeric antibody molecules.

Document AT6 (Cabilly, S. et al. Proc. Natl. Acad. Sci. USA 81:3273-3277 (1984)), describes plasmids that direct the synthesis in E. coli of heavy chains and/or light chains of anti-carcinoembryonic antigen (CEA) antibody. Another plasmid was constructed for expression of a truncated form of heavy chain (Fd') fragment in E. coli extracts, of a portion of the heavy chain with light chain.

Document AL2 (Cabilly, S. et al., European Patent Publication 125023 (published November 14, 1984)) describes chimeric immunoglobulin genes and their presumptive products as well as other modified forms. On pages 21, 28 and 33, cDNA cloning and priming are discussed.

Document AM2 (Boss, M.A. European Patent Application 120694 (published October 3, 1984)), discloses expression in E. coli of non-chimeric immunoglobulin chains with 4-nitrophenyl specificity. There is a broad description of chimeric antibodies but no details (see p. 9).

Document AR7 (Wood, C.R. et al., Nature 314:446 (1985)) describes plasmids that direct the synthesis of mouse anti-NP antibody proteins in yeast. $\mu$ heavy chains appeared to be glycosylated in the yeast cells. When both heavy and light chains were synthesized in the same cell, some of the protein was assembled into functional antibody molecules, as detected by anti-NP binding activity in soluble protein prepared from yeast cells.

Document AS7 (Tan et al., J. Immunol. 135:8564 (1985)) discloses expression of a chimeric human-mouse immunoglobulin genomic gene after transfection into mouse myeloma cells.

Document AT7 (Jones, P.T. et al., Nature 321:522 (1986)) discloses construction and expression of a genomic construct where CDR domains of variable regions from a mouse mAb were used to substitute for

4

the corresponding domains in a human antibody.

Document AR8 (Verhoeyan et al., Science 239:1534 (1988)) discloses that a similar CDR substitution reshaped an antibody against lysozyme.

Document AS8 (Morrison et al. Science 229:1202-1207 (1985)) provides a review of the field.

Document AT8 (Oi, V.T. et al. BioTechniques 4:214-221 (1986)) provides a review of the field. This document also argues that the production of chimeric antibodies from cDNA constructs intra-cellularly in yeast and/or bacteria is not necessarily advantageous.

Document AR9 (Morrison, S.L., Science 239:G28,G48 (1988)) provides a recent review of the field.

Document AN2 (Japanese patent publication No. 61-167699 (published July 29, 1986)) discloses the KM10 mouse mAb.

## SUMMARY OF THE INVENTION

The invention provides an engineered chimeric antibody of desired variable region specificity and constant region properties, produced after gene cloning and expression of light and heavy chains. The chimeric antibody and its derivatives may have applicability in the treatment and diagnosis of human cancer. The cloned immunoglobulin gene products and their derivatives can be produced in mammalian or microbial cells.

The invention provides cDNA sequences coding for immunoglobulin chains comprising a constant human region and a variable, non-human, region. The immunoglobulin chains are both heavy and light.

The invention provides sequences as above, present in recombinant DNA molecules in vehicles such as plasmid vectors, capable of expression in desired prokaryotic or eukaryotic hosts. The invention provides hosts capable of producing, by culture, the chimeric antibodies and methods of using these hosts. The invention also provides individual chimeric immunoglobulin chains, as well as complete assembled molecules having human constant regions and mouse variable regions with specificity for the human tumor cell antigen, wherein both variable regions have the same binding specificity.

Among other immunoglobulin chains and/or molecules provided by the invention are:

1. an antibody with monovalent specificity for a tumor cell antigen, i.e., a complete, functional immunoglobulin molecule comprising:

    (a) two different chimeric heavy chains, one of which comprising a variable region with anti-tumor cell specificity, and

    (b) two different light chains, with the corresponding specificities as the variable regions of the heavy chains. The resulting hetero-bifunctional antibody would exhibit monovalent binding specificity toward human tumor cells.

2. antibody fragments such as Fab, Fab', and F(ab')$_2$.

Genetic sequences, especially cDNA sequences, coding for the aforementioned combinations of chimeric chains are also provided herein.

The invention also provides for a genetic sequence, especially a cDNA sequence, coding for the variable region of desired specificity of an antibody molecule heavy and/or light chain, linked to a sequence coding for a polypeptide different than an immunoglobulin chain (e.g., an enzyme). These sequences can be assembled by the methods of the invention, and expressed to yield mixed-function molecules.

The use of cDNA sequences is particularly advantageous over genomic sequences (which contain introns), in that cDNA sequences can be expressed in bacteria or other hosts which lack appropriate RNA splicing systems.

## BRIEF DESCRIPTION OF THE FIGURES

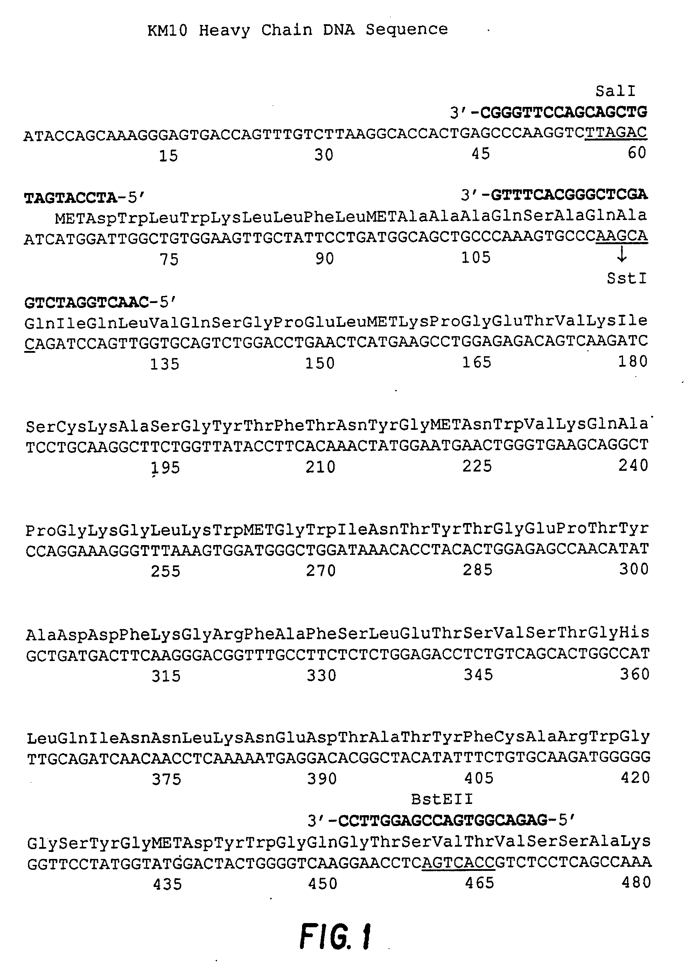

**FIGURE 1.** Nucleotide sequence of the coding strand for the KM10 heavy chain mouse variable region. Shown is the nucleotide sequence from the end of the oligo-dC tail to the $J_H4$ - $C_H1$ junction. Also shown is the amino acid sequence deduced from the nucleotide sequence. Shown in bold are the oligonucleotides used for site directed mutagenesis and the sites at which restriction site modifications were made.

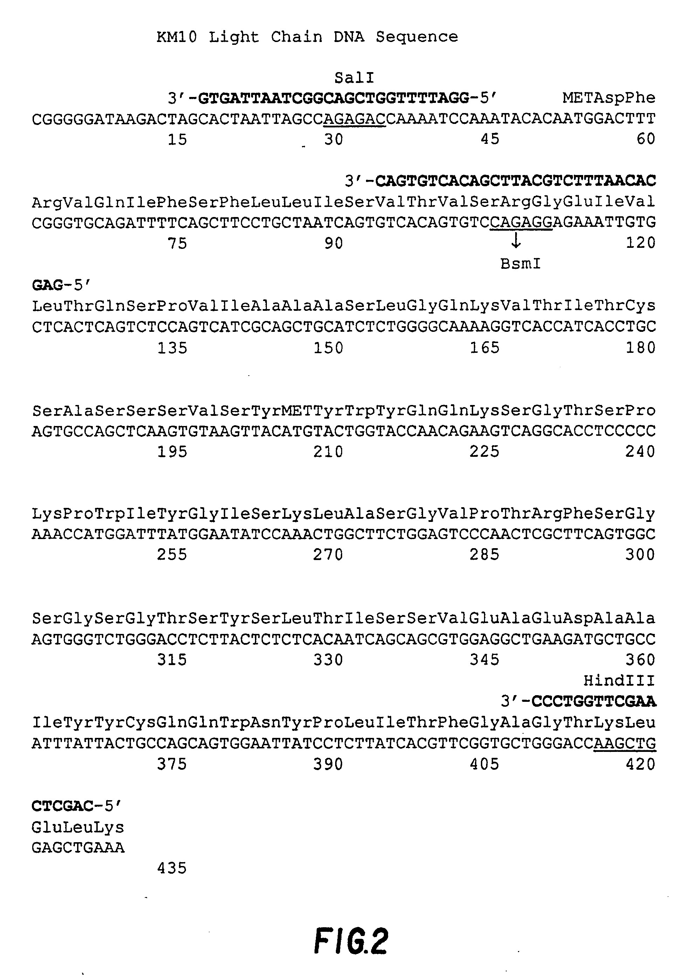

**FIGURE 2.** Nucleotide sequence of the coding strand for the KM10 Kappa mouse variable region. Shown is the nucleotide sequence from the end of the oligo-dC tail to the $J_x5$ - $C_x$ junction. Also shown is the amino acid sequence deduced from the nucleotide sequence. Shown in bold are the oligonucleotides used for site directed mutagenesis and the sites at which restriction site modifications were made.

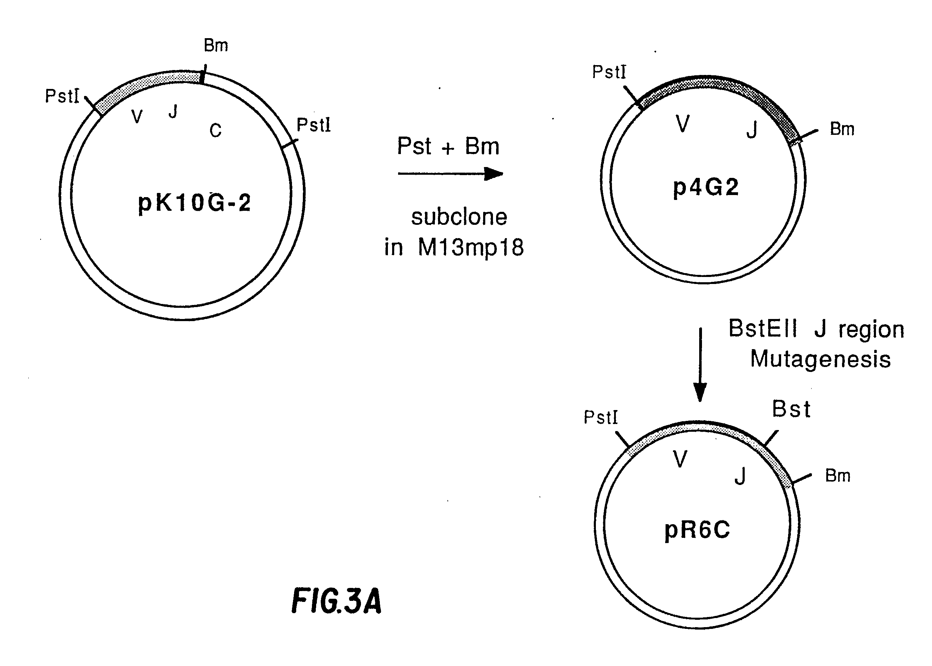

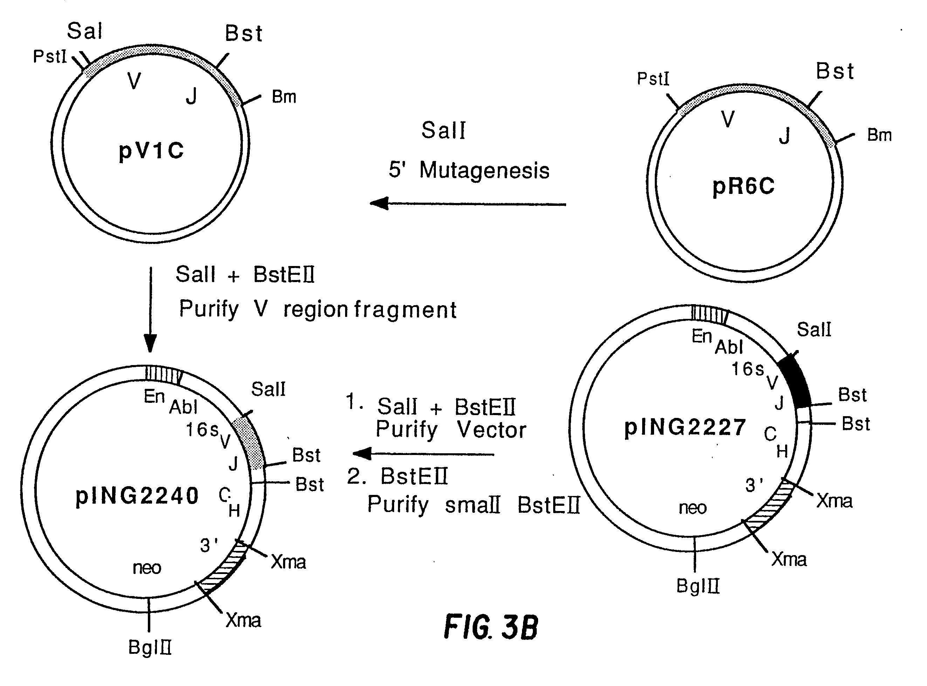

**FIGURE 3.** Construction scheme for the chimeric mouse-human KM10 heavy chain mammalian expression plasmid, pING2240. The variable region for the cDNA clone pM10G-2 was engineered to be compatible with the eucaryotic expression plasmid pING2227. Plasmid pING2227 contains the following gene regulatory elements useful for expression in mammalian cells: 1) the IgG heavy chain enhancer element, 2) an Abelson LTR promoter, 3) the SV40 16S splice site, and 4) the IgG heavy chain polyadenylation signal sequence. It also contains the entire human IgGl constant region from pGMH-6 (Liu, A. Y. et al, Proc. Natl. Acad. Sci., USA 84: 3439 - 3443, 1987). pING2227 contains the neomycin phosphotransferase gene which allows for G418 selection in transfected cells.

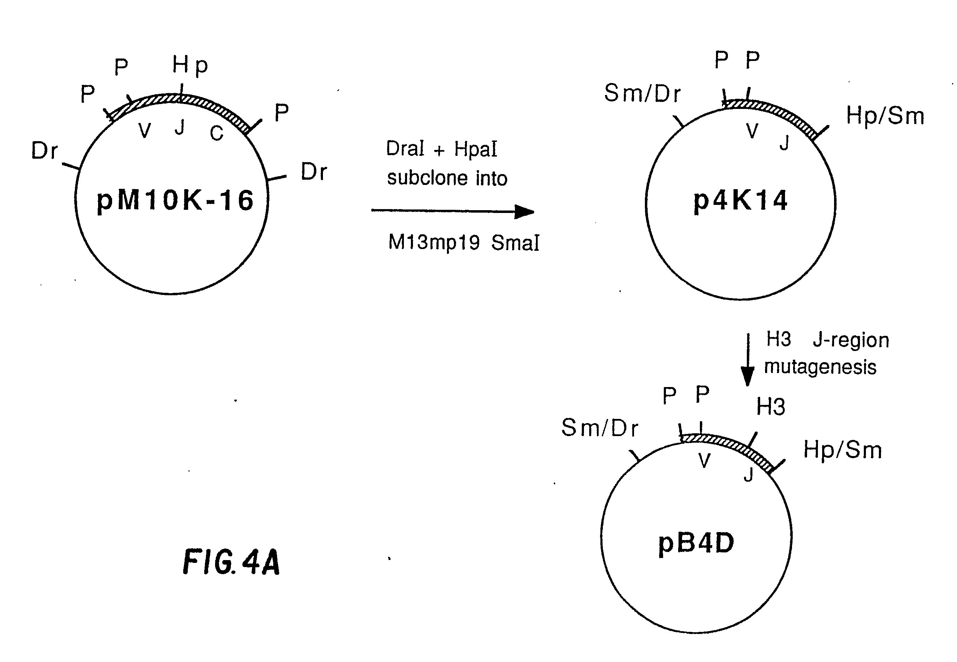

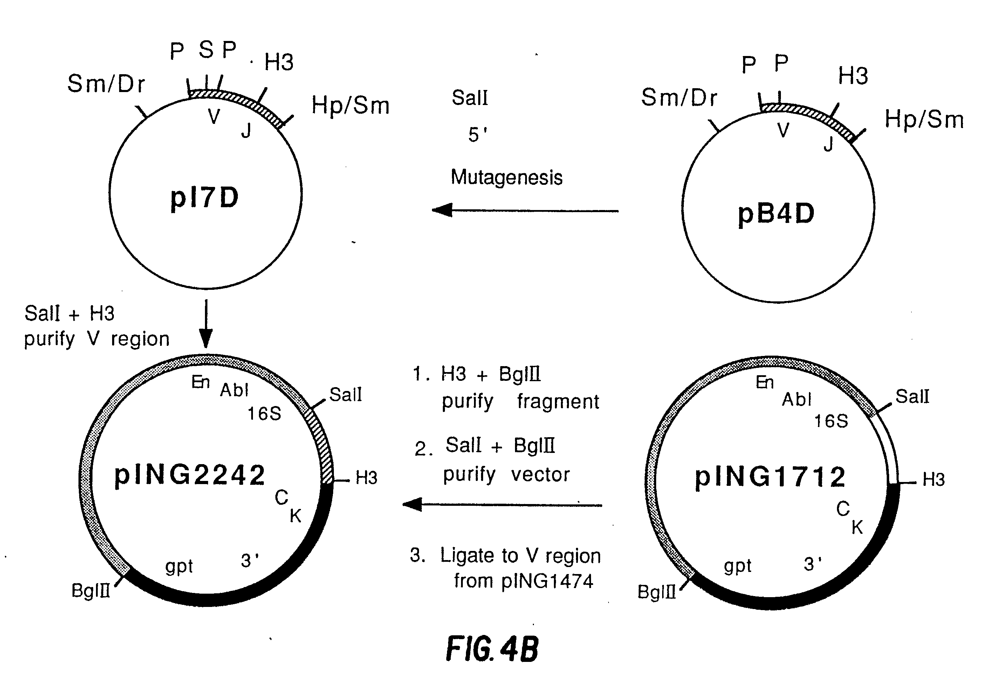

**FIGURE 4.** Construction scheme for the chimeric mouse-human KM10 light chain mammalian expression plasmid pING2242. The variable region from the cDNA clone PM10K-16 was engineered to be compatible with the eucaryotic expression plasmid pING1712. Plasmid pING1712 contains the following gene regulatory elements useful for expression in mammalian cells: 1) the IgH enhancer element, 2) the Abelson LTR promoter, 3) the SV40 16S splice site, and 4) a human x polyadenylation signal sequence. It also contains the entire human x constant region, (Liu A.Y., et al. supra) and the GPT gene which allows for mycophenolic acid resistance in transfected cells.

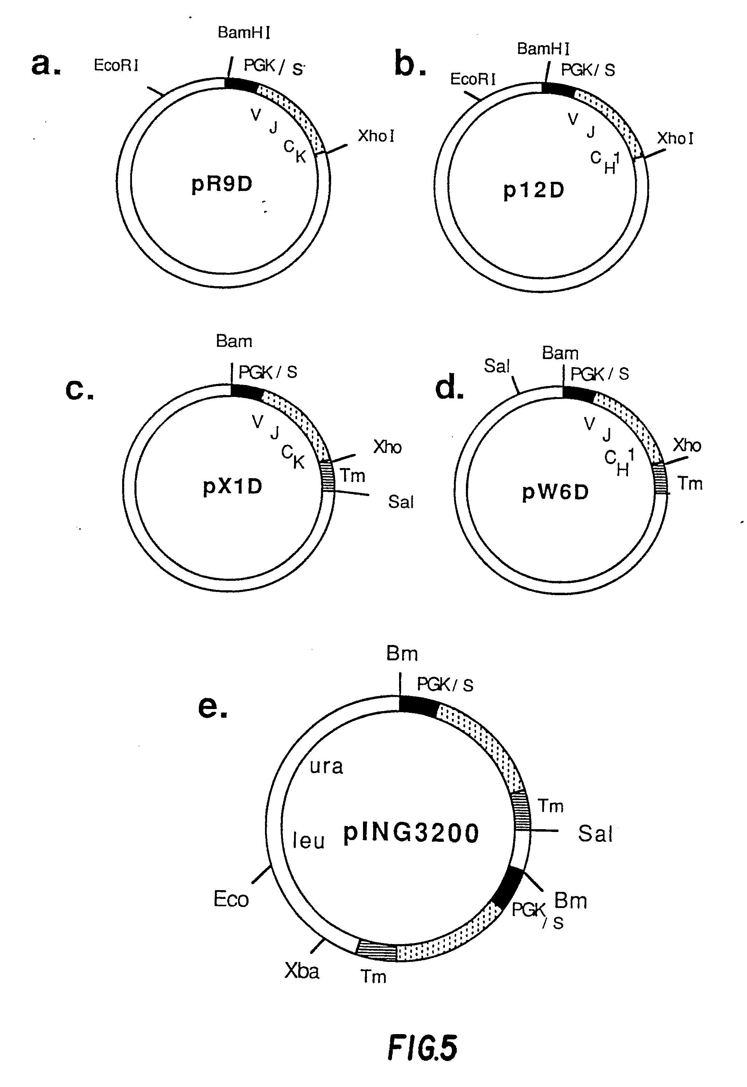

**FIGURE 5.** Yeast expression plasmids for Fab expression. Shown are: (a) the yeast expression plasmid containing KM10 chimeric light chain gene fused to the yeast PGK promoter, invertase signal sequence and PGK polyadenylation signal; (b) the similar yeast plasmid containing the Fd gene; (c) the yeast expression plasmid containing the light chain promoter/leader fusion with PGK transcription termination signal; (d) similar yeast plasmid containing the Fd gene; and (e) the final 2 gene yeast expression plasmid pING3200.

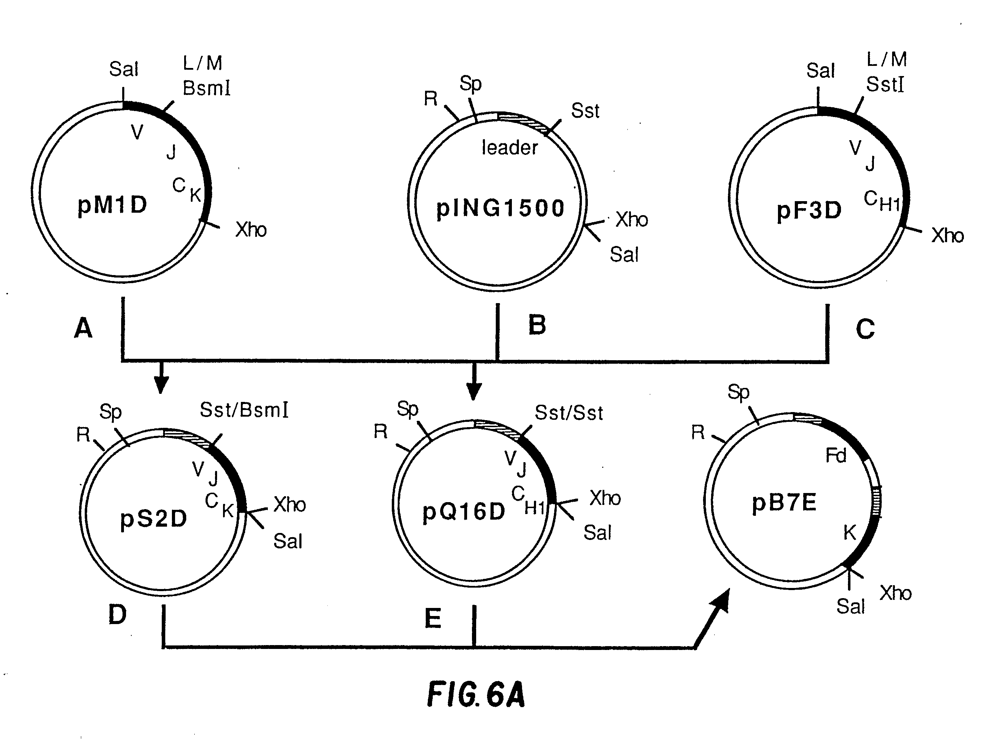

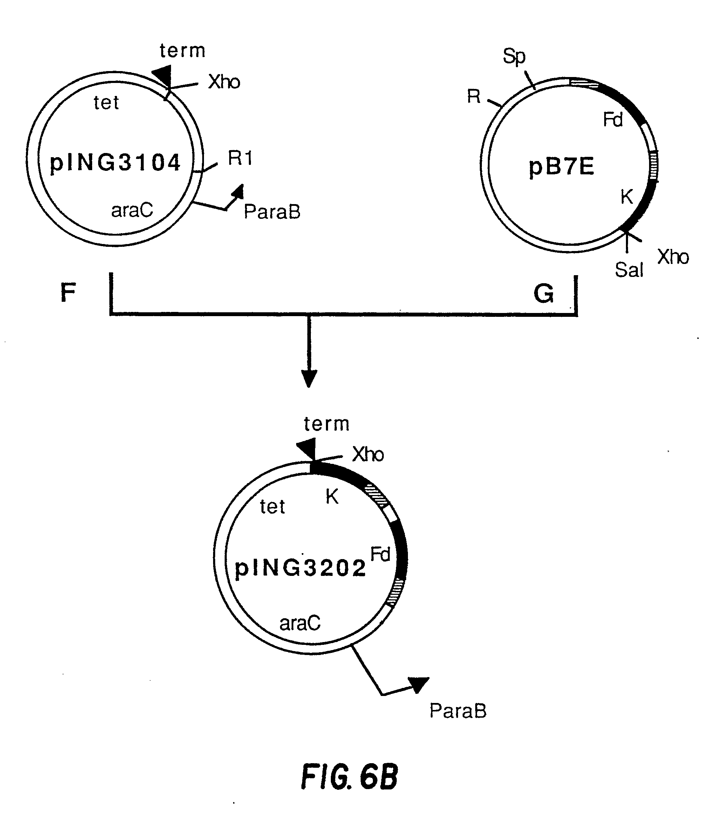

**FIGURE 6.** Construction scheme for the bacterial chimeric KM10 Fab expression plasmid pING3202. Plasmid pING3202 contains the following elements useful for expression in E. coli: 1) the araC gene, 2) the inducible araB promoter, 3) the dicistronic Fd and x KM10 genes fused to the pelB leader sequence, 4) the trpA transcription termination sequence, and 5) the tet$^R$ gene, useful for selection in E. coli.

## DESCRIPTION OF THE PREFERRED EMBODIMENTS

## GENETIC PROCESSES AND PRODUCTS

The invention provides an antibody that can be used for the treatment and diagnosis of human carcinoma, either alone or in combination with other reagents. The antigen is that bound by the mAb designated KM10.

The method of production combines five elements:

1. Isolation of messenger RNA (mRNA) from the mouse B cell hybridoma line producing the mAb, cloning and cDNA production therefrom;

2. Preparation of a full length cDNA library from purified mRNA from which the appropriate variable region gene segments of the light and heavy chain genes can be (i) identified with appropriate probes, (ii) sequenced, and (iii) made compatible with a constant gene segment.

3. Preparation of constant region gene segment modules by cDNA preparation and cloning.

4. Construction of complete heavy or light chain coding sequences by linkage of the cloned specific immunoglobulin variable region gene segments described in 2 above to cloned human constant region gene segment modules described in 3.

5. Expression and production of chimeric light and heavy chains in selected hosts, including prokaryotic and eukaryotic cells.

One common feature of all immunoglobulin light and heavy chain genes and the encoded messenger RNAs is the so-called J region. Heavy and light chain J regions have different sequences, but a high degree of sequence homology exists (greater than 80%) among each group, especially near the constant region. This homology is exploited in this invention, and consensus sequences of light and heavy chain J regions were used to design oligonucleotides for use as primers or probes for introducing useful restriction sites into the J region for subsequent linkage of variable region segments to human constant region segments.

Constant region cDNA module vectors prepared from human cells and modified by site-directed mutagenesis to place a restriction site at the analogous position in the human sequence were used. For example, one can clone the complete human $x$ light chain C region and the complete human $\gamma_1$ C region. An alternative method utilizing genomic C region clones as the source for C region module vectors would not allow these genes to be expressed in systems such as bacteria where enzymes needed to remove intervening sequences are absent.

Cloned V region segments are excised and ligated to light or heavy chain C region module vectors. In addition, the human $\gamma1$ region can be modified by introducing a termination codon thereby generating a gene sequence which encodes only a portion of the heavy chain, such as the segment found in an Fab molecule.

The coding sequences with linked V and C regions (or portions thereof) are then transferred into appropriate expression vehicles for expression in appropriate hosts, prokaryotic or eukaryotic. Linked means in-frame joining of coding sequences to derive a continuously translatable gene sequence without alterations or interruptions of the triplet reading frame. Expression vehicles include plasmids or other vectors. Preferred among these are vehicles carrying a functionally complete human constant heavy or light chain sequence having appropriate restriction sites engineered so that any variable heavy or light chain sequence with appropriate cohesive ends can be easily inserted thereinto. Human constant heavy or light chain sequence-containing vehicles are thus an important embodiment of the invention. These vehicles can be used as intermediates for the expression of any desired complete heavy or light chain in any appropriate host.

One preferred host is yeast. Yeast provides substantial advantages for the production of immunoglobulin light and heavy chains. Yeasts carry out post-translational peptide modifications including glycosylation. A number of recombinant DNA strategies now exist which utilize strong promoter sequences and high copy number plasmids which can be used for production of the desired proteins in yeast. Yeast recognizes leader sequences of cloned mammalian gene products and secretes peptides bearing leader sequences (i.e. prepeptides) (Hitzman et al., 11th International Conference on Yeast, Genetics and Molecular Biology, Montpellier, France, September 13-17, 1982).

Yeast gene expression systems can be routinely evaluated for the levels of production, secretion and the stability of chimeric heavy and light chain proteins and assembled chimeric antibodies. Any of a series of yeast gene expression systems incorporating promoter and termination elements from the actively expressed genes coding for glycolytic enzymes produced in large quantities when yeasts are grown in media rich in glucose can be utilized. Known glycolytic genes can also provide very efficient transcription control signals. For example, the promoter and terminator signals of the iso-I-cytochrome C (CYC-1) gene can be utilized. A number of approaches may be taken for evaluating optimal expression plasmids for the expression of cloned immunoglobulin cDNAs in yeast.

Bacterial strains may also be utilized as transformation hosts for the production of antibody molecules or antibody fragments described by this invention. E. coli K12 strains such as E. Coli W3110 (ATCC 27325) and other enterobacteria such as Salmonella typhimurium or Serratia marcescens, and various Pseudomonas species may be used.

Plasmid vectors containing replicon and control sequences which are derived from species compatible with a host cell are used in connection with these bacterial hosts. The vector carries a replication site, as well as specific genes which are capable of providing phenotypic selection in transformed cells. A number of approaches may be taken for evaluating the expression plasmids for the production of chimeric antibodies or antibody chains encoded by the cloned immunoglobulin cDNAs in bacteria.

Other preferred hosts are mammalian cells, grown in vitro or in vivo. Mammalian cells provide posttranslational modifications to immunoglobulin protein molecules including leader peptide removal, folding and assembly of heavy and light chains, glycosylation of the antibody molecules, and secretion of functional antibody protein. Mammalian cells which may be useful as hosts for the production of antibody proteins include cells of lymphoid origin, such as the hybridoma Sp2/0-Ag14 (ATCC CRL 1581) or the myeloma P3X63Ag8 (ATCC TIB 9), and its derivatives. Others include cells of fibroblast origin, such as Vero (ATCC CRL 81) or CHO- K1 (ATCC CRL 61).

Many vector systems are available for the expression of cloned heavy chain (H) and light chain (L) genes in mammalian cells. Different approaches can be followed to obtain complete $H_2L_2$ antibodies. It is possible to co-express light and heavy chains in the same cells to achieve intracellular association and linkage of heavy and light chains into complete tetrameric $H_2L_2$ antibodies. The co-expression can occur by using either the same or different plasmids in the same host. Genes for both heavy and light chains can be placed into the same plasmid, which is then transfected into cells, thereby selecting directly for cells that express both chains. Alternatively, cells may be transfected first with a plasmid encoding one chain, for example light chain, followed by transfection of the resulting cell line with a heavy chain plasmid containing a second selectable marker. Cell lines producing $H_2L_2$ molecules via either route could be transfected with plasmids encoding additional copies of light, heavy, or light plus heavy chains in conjunction with additional selectable markers to generate cell lines with enhanced properties, such as higher production of assembled ($H_2L_2$) antibody molecules or enhanced stability of the transfected cell lines.

POLYPEPTIDE PRODUCTS

The invention provides "chimeric" immunoglobulin chains, either heavy or light with specificity toward human tumor cells. A chimeric chain contains a constant region substantially similar to that present in a natural human immunoglobulin, and a variable region having the desired anti-tumor specificity of the invention. The invention also provides immunoglobulin molecules having heavy and light chains associated so that the overall molecule exhibits the desired binding and recognition properties. Various types of immunoglobulin molecules are provided: monovalent, divalent, or molecules with the invention's variable binding domains attached to moieties carrying desired functions. This invention also provides for fragments of chimeric immunoglobulin molecules such as Fab, Fab', or F(ab')$_2$ molecules or those proteins coded by truncated genes to yield molecular species functionally resembling these fragments.

Antibodies having chimeric heavy chains and light chains of the same or different variable region binding specificity, can be prepared by appropriate association of the needed polypeptide chains. These chains are individually prepared by the modular assembly methods of the invention.

USES

The antibodies of this invention can be tested for therapeutic purposes by themselves, for example, acting via ADCC, or coupled to toxins or therapeutic moieties, such as ricin, radio-nuclides, drugs, etc. in the treatment of human cancer. The antibodies may be advantageously utilized in combination with factors, such as lymphokines, colony stimulating factors, and the like, which increase the number or activity of ADCC effector cells.

The antibodies of the invention having human constant region are utilized for passive immunization, especially in humans, with less untoward immune reactions such as serum sickness or anaphylactic shock, as compared to whole mouse antibodies. The antibodies can also be utilized in immunodiagnostic assays and kits in detectably labelled form (e.g., with enzymes, $^{125}$I, $^{14}$C, fluorescent labels, etc.), or in immobilized form (on polymeric tubes, beads, etc.). They can also be utilized in labelled form for in vivo imaging, wherein the label is a radioactive emitter, or an nuclear magnetic resonance contrasting agent such as a heavy metal nucleus, or an X-ray contrasting agent, such as a heavy metal. The antibodies can also be used for in vitro localization of the recognized tumor cell antigen by appropriate labelling.

Mixed antibody-enzyme molecules can be used for immunodiagnostic methods, such as ELISA. Mixed antibody-peptide effector conjugates can be used for targeted delivery of the effector moiety with a high degree of efficacy and specificity.

Specifically, the chimeric antibodies of this invention can be used for any and all uses in which the murine KM10 mAb can be used, with the obvious advantage that the chimeric antibodies are more compatible with the human body.

Having now generally described the invention, the same will be further understood by reference to certain specific examples which are included herein for purposes of illustration only and are not intended to be limiting unless otherwise specified.

EXAMPLE 1

Preparation of Monoclonal Antibody KM10

1. Preparation of immunogen

The low-differentiation human gastric adenoma-derived cell line (MKN-45) was disrupted by ultra sonication. After centrifugation (15,000 x g, 30 min), the precipitate was discarded. The supernatant was applied to a Sepharose 4b gel filtration column. The fractions ranging in molecular weight from 700 kD to 1,500 kD were recovered and mixed with Freund's complete adjuvant. The mixture was administered intraperitoneally to BALB/c mice. The mice were immunized once a week for 5 weeks. Four days after the final immunization, the spleen was obtained from the mouse to be used for the cell fusion.

## 2. Cell fusion and cloning

(a) Description of the Method

The above-mentioned mouse splenic cells and the mouse myeloma P3U1 (see. Curr. Top. Microbiol. Immunol. 81: 1 (1978)) were mixed in the ratio of 4:1 and a fusion reaction was carried out for 2 minutes using 42.6% polyethylene glycol (average molecular weight.. 1,000) by a partial modification of the method of Kohler et al. (Immunological Methods, Academic Press, New York, p. 391 (1979)).

The treated cells were inoculated into 96-well microplates and cultured in HAT medium (see below) for 10 to 14 days; thereafter, they were transferred to HT medium (see below). After their growth became sufficient to enable cultivation in a flask (25 cm$^2$), they were cultured in D-MEM medium (see below). The antibody titer of the culture supernatant of each well in which proliferation occurred was determined by the ELISA method, and cloning of the desired hybridoma was conducted by limiting dilution analysis.

Mouse peritoneal exudate cells (25,000/well) were used as a feeder layer. The hybridomas were then diluted with D-MEM medium to 10, 5, 2.5, 1 cell/0. 1 ml, and 0.1 ml of each of these dilutions was inoculated into wells of the microtiter plate and cultured. Four days later, 0.1 ml of D-MEM medium was added; thereafter one half of the medium was exchanged at intervals of 4 to 7 days. Within 10 to 20 days after the initiation of cultivation, visually recognizable colonies were formed and clones were obtained.

(b) Selection Media

Dulbecco's modified Eagle medium (D-MEM), produced by Nissui Seiyaku Co. was the basal medium used.

HAT medium was prepared by supplementing D-MEM with the following additives: Horse serum (10%, Flow Laboratories), L-glutamine (300 mg/L), sodium pyruvate (100 mg/L), penicillin (100 IU/ml), streptomycin (100 μg/ml), glucose (3.5 g/L), NaHCO$_3$ (3.7 g/L), hypoxanthine (1x10$^{-4}$ M), aminopterin (4X10$^{-7}$ M), thymidine (1.6x10$^{-5}$ M).

HT medium contained the same additives as HAT medium with the exception that aminopterin was not included.

## 3. Method of screening

The obtained hybridomas were screened for clones that produce the desired mAb as follows:

(a) Description of the method

The method of ELISA was performed as follows. The hybridoma culture supernatant was added to wells of a microplate coated with an antigen (one of various established cancer cell lines, partially purified cancer-associated antigens or normal cells), and then the plate was incubated at 37° C for 1 hr. After washing the microplate, peroxidase-labeled anti-mouse immunoglobulin (IgG + IgA + IgM) rabbit antibodies were added and allowed to incubate at 37° C for 1 hr. After washing to remove excess labeled antibodies, 0-phenylenediamine solution was added as substrate, and the enzymatic reaction carried out at room temperature for 30 min. The reaction was stopped by adding 2M sulfuric acid and the absorbance at 490 nm was determined. Reactivities with various cells were examined by this method. Cross-reactivity with leukocytes was determined by using an antibody labeled with β-galactosidase, cross-reactions with red blood cell were determined by the PHA method using a mixture of human type A, B and 0 red blood cells.

9

(b) Flow sheet of the screening

Primary screening was by ELISA using the tumor target cell, MKN-45 and as a control, fetal lung-derived fibroblast (Flow-2000); wells that were positive for binding to MKN-45 and negative for binding to Flow-2000 were selected. Secondary screening involved other normal tissue-derived cell lines, leukocytes and red blood cell; wells that were negative for all these normal cell types were selected.

For a tertiary round of screening, hybridomas selected in the secondary screening were cloned 2 to 3 times. Culture supernatants were examined for reactivity with various cancer-derived established cell lines.

The hybridoma selected in the tertiary screening was named KM10.

The hybridoma producing mAb KM10 was deposited at the Institute for Fermentation, Osaka (IFO) in Osaka, Japan on March 24, 1989 under accession number IFO 50187.

## 4. Characterization of mAb KM10.

This antibody is of isotype IgG1 and binds to an antigen which is expressed on the surface of cells from many human carcinomas, including colon, stomach, pancreas and esophagus; the antigen is only present at trace levels in normal adult cells.

## EXAMPLE 2

## A Chimeric Mouse-Human Immunoglobulin with Human Tumor Specificity Produced from Mammalian Cells

KM10 mAb was obtained from a mouse which had been immunized with cells from a human colon carcinoma, after which spleen cells were hybridized with NS-1 mouse myeloma cells. The antibody binds to an antigen which is expressed on the surface of cells from many human carcinomas, including colon, stomach, pancreas and esophagus, while the antigen is only present at trace levels in normal adult cells. MAb KM10 is of isotype IgG1.

## 1. Recombinant Plasmid and Bacteriophage DNAs

Oligo-dG tailed pBR322, pUC18, pUC19, M13mp18, and M13mp19 were purchased from BRL (Gaithersburg, Maryland). DNA manipulations involving purification of plasmid DNA by buoyant density centrifugation, restriction endonuclease digestion, purification of DNA fragments by agarose gel electrophoresis, ligation and transformation of E. coli were as described by Maniatis, T., et al., Molecular Cloning: A Laboratory Manual, (1982), or other standard procedures. Restriction endonucleases and other DNA/RNA modifying enzymes were purchased from Boehringer-Mannheim (Indianapolis, Indiana), BRL, and New England Biolabs (Beverly, Massachusetts).

## 2. RNA Purification and cDNA Library Construction

One liter of KM10 hybridoma cells at approximately $1 \times 10^6$ cells/ml were collected by centrifugation and washed in 100 ml of PBS (8g NaCl, 0.2g $KH_2PO_4$, 1.15g $Na_2HPO_4$,and 0.2g KCl per liter). The cells were centrifuged again and the cell pellet was suspended in a solution of guanidine thiocyanate, and total cellular RNA was prepared from tissue culture cells by the method described in Maniatis, T., et al., supra. Preparation of poly(A)$^+$ RNA was as described by Maniatis, T., et al., supra.

Oligo-dT primed cDNA libraries were prepared from poly(A)$^+$ RNA by the method of Gubler et al., Gene 25:263 (1983). The cDNA was dC-tailed with terminal deoxynucleotide transferase and annealed to dG-tailed pBR322. cDNA libraries were screened by hybridization (Maniatis, T., supra) with $^{32}$P-labelled, nick translated DNA fragments, i.e., for kappa clones with a mouse $C_x$ region probe and for heavy chain clones with a mouse IgG1 constant region probe.

The light and heavy chain V region fragments from the full length cDNA clones, pM10K-16 and pM10G-2 respectively, were inserted into M13 bacteriophage vectors for nucleotide sequence analysis. The complete nucleotide sequences of the variable region of these clones were determined (FIGURES 1 and 2) by the dideoxy chain termination method. These sequences predict V region amino acid compositions that agree well with the observed compositions, and predict peptide- sequences which have been verified by direct amino acid sequencing of portions of the V regions.

The nucleotide sequences of the cDNA clones show that they are immunoglobulin V region clones as they contain amino acid residues diagnostic of V domains (Kabat et al., Sequences of Proteins of Immunological Interest; U.S. Dept of HHS, 1983).

The KM10 $V_H$ belongs to subgroup II. The KM10 $V_H$ has the $J_H4$ sequence and the KM10 $V_x$ has the $J_x5$ sequence.

### 3. Construction of Chimeric Expression Plasmids

Expression vectors appropriate for the insertion of $V_H$ and $V_L$ gene modules to obtain expression of chimeric KM10 were constructed. The light chain vector pING1712 was made by first making a plasmid DNA containing a test chimeric light chain gene (pING2122) and adding a mouse Abelson LTR promoter, a splice region, and a mouse genomic kappa region 3' to the polyadenylation signal. The heavy chain mouse enhancer 0.7kb XbaI to EcoRI fragment from M13 M8alphaRX12 (Robinson, R.R., et al., PCT US86/02269) was inserted into XbaI plus EcoRI cut M13mp19. The enhancer-containing HindIII to BglII fragment was inserted into the BglII to HindIII region of pSH6, an E. coli recombinant plasmid DNA that contains unique XhoI, BglII, and HindIII sites, in that order. The enhancer-containing XbaI to XhoI fragment was then inserted into the enhancer XbaI to XhoI region of pING2121b, an expression plasmid identical to pING2108b (Liu, A.Y., et al., J. Immunology 139:3521 (1987)) except that the L6 VL region (Liu, A.Y., et al., Proc. Natl. Acad. Sci. USA 84:3439 (1987)) was used in its construction instead of the 2H7 VL region. The resulting plasmid was pING2122.

The mouse Abelson virus LTR was obtained from pelin2 (provided by Dr. Owen Witte, U.C.L.A.). pelin2 contains the p120 viral 3' LTR (Reddy, E.P., et al., Proc. Natl. Acad. Sci. USA 80:3623 (1983)) except that the BglII site at viral position 4623 has been modified by insertion of the EcoRI oligonucleotide linker GGATTCC. The 0.8kb EcoRI to KpnI fragment of pelin2 containing the p120 3' LTR promoter was inserted into KpnI plus EcoRI cut pUC18. The LTR was excised as an EcoRI to SalI fragment and ligated to EcoRI plus SalI cut pING2122, resulting in a plasmid where the LTR promoter is adjacent to the L6 light chain gene (pING2126). An XhoI to SalI fragment containing SV40 16S splice donor and acceptor sites was excised from pUC12/pL1 (Robinson et al., PCT US86/02269) and inserted into the SalI site of pING2126, screening for the orientation where the splice donor was between the LTR and the light chain gene (pING2133).

The polyadenylation/transcription termination region of the kappa expression vector was also modified. The first step was the HindIII digestion and religation of plasmid pING2121a, which is identical to pING2108a (Liu, A.Y., et al., J. Immunology 139:3521 (1987)) except that the L6 VL was used in its construction instead of the 2H7 VL, to form pING2121a-deltaH. The 1.1kb BglII to BamHI fragment of mouse genomic DNA distal to the polyadenylation site (Xu, M., et al., J. Biol. Chem. 261:3838 (1986) was isolated from pS107A (provided by Dr. Randolph Wall, U.C.L.A.) and inserted into the BamHI site of pING2121a-deltaH, screening for the orientation homologous to the native gene. The 3.3kb BglII to SstI fragment containing this modified 3' region was ligated to the 5.2kb BglII to SstI fragment of pING2121b to form pING1703. The BglII to SalI fragment of pING1703 with the modified 3' region and chimeric kappa coding sequence was ligated to the large BglII to SalI fragment of pING2133, resulting in the 9.1kb kappa expression vector pING1712 shown in Figure 4. The Abelson LTR promoter was also used in the chimeric heavy chain expression vector pING1714. pING2111 (Robinson, R.R., et al., PCT US 86/02269) was modified by the insertion of an AatII oligonucleotide linker at the XbaI site, followed by AatII cleavage and religation to form pING1707. The AatII to SalI fragment containing the Abelson LTR promoter was excised from pING2133 and ligated to the large AatII to SalI fragment of pING1707 to form pING1711. The heavy chain enhancer was deleted from pING1711 by EcoRI digestion, T4 polymerase treatment, ligation to AatII oligonucleotide linker, and cleavage and religation with AatII to form the 7.7kb expression vector pING1714.

A similar plasmid, pING2227, contains two additional regulatory elements, the IgH enhancer and the human genomic IgG polyadenylation sequence. pING2227 is identical to pING1712 in the region from BglII to SalI containing the IgH enhancer the Abelson LTR promoter, and the 16S slice donor and acceptor sites. The human genomic IgG 3' end sequence was ligated as an 1185 bp XmaIII DNA fragment into an XmaIII site located 6 bp past the termination codon for the heavy chain gene in pING1714. The 1300 bp XmaIII fragment containing the genomic gamma 3' end was isolated from a derivative of pHG3A (Ellison et al., Nucleic Acids Research 10:4071 (1982)).

4. Construction of a KM10 Heavy and Light Chain Expression Plasmid

The cDNA clone containing the KM10 heavy chain, pK10G, was adapted for mammalian expression by introducing convenient restriction endonucleases sites by site directed mutagenesis (Kramer, W., et al., Nucl. Acids Res. 12:9441-9456 (1984)) into appropriate M13 subclones, Figure 3. Oligonucleotides were synthesized on a Cyclone DNA synthesizer, New Brunswick Scientific Co., and purified by acrylamide gel electrophoresis. The J-region mutagenesis primer 5'-GAGACGGTGACCGAGGTTCC-3' was used to insert a BstEII site into the M13 subclone p4G2, and the oligonucleotide 5'-ATCCATGATGTCGACGACCTTGGGC-3' was used to insert a SalI restriction site into pR6C upstream of the initiation codon ATG. The restriction fragment containing the KM10 heavy chain V-region bounded by SalI and BstEII was then cloned into the expression vector pING2227.

The cDNA clone containing the KM10 light chain, pM10K-16, was adapted for mammalian expression in a similar way, Figure 4. The J-region mutagenesis primer 5'-CAGCTCAAGCTTGGTCCC-3' was used to insert a HindIII site into the M13 subclone p4K14, and the oligonucleotide 5'-GGATTTTGGTCGACGGCTAATTAGTG-3' was used to insert a SalI restriction site into p4BD upstream of the initiation codon ATG. The restriction fragment containing the KM10 light chain V-region bounded by SalI and HindIII was then cloned into the expression vector pING1712.

5. Stable Transfection of Mouse Lymphoid Cells for the Production of Chimeric Antibody.

The cell line Sp2/0 (American Type Culture Collection #CRL1581) was grown in D-MEM (see above) supplemented to 4.5g/l glucose, and additionally containing 10% fetal bovine serum.

The electroporation method of Potter, H., et al. (Proc. Natl. Acad. Sci. USA 81:7161 (1984)) was used. After transfection, cells were allowed to recover in complete D-MEM for 24 hours, and then seeded at 10,000 to 50,000 cells per well in 96-well culture plates in the presence of selective medium. G418 (GIBCO) selection was at 0.8 mg/ml, and mycophenolic acid (Calbiochem) was at 6 μg/ml plus 0.25 mg/ml xanthine. The electroporation technique gave a transfection frequency of 1-10 x $10^{-5}$ for the Sp2/0 cells.

The chimeric KM10 light chain expression plasmid pING2242 was linearized by digestion with PvuI restriction endonuclease and transfected into Sp2/0 cells, giving mycophenolic acid resistant clones which were screened for light chain synthesis. The best producer after outgrowth and subsequent subcloning, was transfected with PvuI-linearized pING2240, the expression plasmid containing the chimeric KM10 heavy chain gene. After selection with G418, the clone producing the most light plus heavy chain, Sp2/0-22426G2-22401C4 (ATCC Accession #HB 10131), secreted antibody at approximately 21 μg/ml.

6. Purification of Chimeric KM10 Antibody Secreted in Tissue Culture.

Sp2/0-22426G2-22401C4 cells (ATCC Accession #HB 10131) were grown in culture medium HB101 (Hana Biologics) + 1% Fetal Bovine Serum, supplemented with 10mM HEPES, 1x Glutamine-Pen-Strep (Irvine Scientific #9316). The spent medium·was centrifuged at about 14,000 x g for 20 minutes and the supernatant was filtered through a 0.45 μm Millipore nitrocellulose membrane filter and stored frozen. The antibody content was measured by ELISA. Approximately 15.5L of cell culture supernatant were concentrated 10-fold over a S10Y30 cartridge using DC-10 concentrator (Amicon Corp.). Supernatant containing about 80 mg of antibody was loaded onto a 100 ml Protein A-column (MabLab, Oros) in 1.5 M NaCl, pH 8.4. The KM10 antibody was eluted with a pH gradient (pH 2-9) and was found to elute between pH 3.5 - 4.0. Fractions containing antibody (70% yield) were combined and concentrated 18-fold by ultrafiltration (YM30 membrane, stirred cell, Amicon Corp.), diluted 20-fold with PBS, reconcentrated 5-fold, diluted 1.5-fold with PBS, and finally reconcentrated 10 fold. The antibody was stored in 1.5 ml aliquots at -20°C.

7. Analysis of the Chimeric KM10 Antibody

(a) Inhibition of Binding:

The mouse KM10 mAb and chimeric KM10 antibodies were compared in a binding inhibition assay. Such inhibition assays are used to establish the identity of recognition of antigen. Mouse KM10 mAb was labeled with $^{125}$I; purified unlabeled chimeric KM10 and mouse KM10 antibodies were examined for their ability to inhibit the binding of radio-labeled KM10 antibody to target cells (LS174T colon tumor). The chimeric KM10 and mouse KM10 antibodies were identical in inhibition of the binding of labeled KM10 antibody to LS174T tumor cells (Table 1).

Table 1.

| Inhibition of Binding of KM10 Antibody to LS174T Tumor Cells. | | | |
|---|---|---|---|
| | % Inhibition by Competing Antibody:[a] | | |
| Antibody Concentration μg/ml | Chimeric KM10 | Mouse KM10 | Human IgG[b] |
| 0.15 | 2 | 2 | -8 |
| 0.45 | 9 | 14 | 2 |
| 1.35 | 9 | 32 | 18 |
| 4.04 | 42 | 46 | 6 |
| 12.1 | 63 | 59 | 14 |
| 36.4 | 74 | 80 | -7 |
| 109 | 75 | 72 | -22 |

[a] $^{125}$I-labeled KM10 antibody was incubated with LS174T tumor cells in the presence of the competing antibody at 4° C. Cells were washed free of unbound antibody, and cell-bound radioactivity was used to de ermine the % inhibition of binding.
[b] Human IgG is used as a nonspecific antibody control.

(b) Functional Assays:

A comparison was made between the ability of the chimeric KM10 and the mouse KM10 antibodies to lyse human tumor cells in the presence of human peripheral blood leukocytes as ADCC effector cells, or human serum as complement for CDC. Table 2 shows that the chimeric KM10 antibody was capable of mediating ADCC while the mouse antibody was not. Neither mouse nor chimeric KM10 were able to detectably lyse target LS174T cells in CDC in the presence of human serum.

EP 0 404 003 A2

Table 2.

| Antibody-Dependent Cellular Cytotoxicity Mediated by Chimeric KM10 Antibody[a] | | |
|---|---|---|
| | % Cytolysis Mediated by: | |
| Antibody Concentration $\mu$g/ml | Chimeric KM10 | Mouse KM10 |
| 50. | 80 | 26 |
| 5. | 61 | 20 |
| .5 | 39 | 22 |
| .05 | 27 | 21 |
| .005 | 23 | 21 |
| .0005 | 23 | 21 |
| 0 | 24 | 22 |

[a] - LS174T tumor cells were labeled with $^{51}$Cr, washed, and incubated with freshly isolated peripheral blood leukocytes in the presence of 17% human serum at a ratio of 50 leukocytes per tumor cell for 4 ho rs at 37° C. The amount of $^{51}$Cr released into the medium was used to calculate the % cytolysis as compared to cells lysed by the addition of 1% NP40.

## EXAMPLE 3

A Chimeric Mouse-Human Fab with Human Tumor Cell Specificity Produced in Yeast

Yeast cells are capable of expressing and secreting foreign proteins. In this example, yeast cells serve as hosts for the production of mouse-human chimeric Fab. This reagent may prove useful in cancer diagnosis by in vivo imaging of appropriately labeled Fab, and in cancer therapy by administration of the Fab as a drug, radionuclide, or toxin immunoconjugate.

### 1. Yeast Strains and Growth Conditions

Saccharomyces cerevisiae strain PS6 (ura3 leu2 MATa) was developed at INGENE and used as a host for yeast transformations performed as described by Ito et al., J. Bacteriol. 153:163-168 (1983). Yeast transformants were selected on SD agar (2% glucose, 0.67% yeast nitrogen base, 2% agar) and grown in SD broth buffered with 50 mM sodium succinate, pH5.5.

### 2. In Vitro Mutagenesis

Site directed in vitro mutagenesis was performed as described by Kramer et al., supra, to introduce a Bsml restriction site into the KM10 x light chain cDNA sequence (Figure 1) at the junction of the leader peptide and mature coding region with the oligonucleotide primer 5'-GAGCACAATTTCTGCATTCGACACTGTGAC-3'. An Sst1 site was similarly introduced at the junction of the leader peptide and mature coding region of the KM10 heavy chain with the oligonucleotide primer 5'-

14

CAACTGGATCTGAGCTCGGGCACTTTG-3' (Figure 2).

### 3. Construction of Yeast Expression Plasmids Containing Antibody Genes.

The gene sequences encoding the mature form of the light chain V region of KM10 and containing a HindIII site in the J region (as described in Example 1) and a BsmI site introduced at the signal sequence processing site was fused to the human $C_x$ region by cloning a SalI-HindII fragment containing V into a vector containing the gene sequences encoding human $C_x$ (pING1460), generating the KM10 chimeric light chain plasmid pM1D (see Figure 6).

The mature chimeric KM10 light chain gene from pM1D was next fused to the gene sequence encoding the yeast invertase signal sequence (Taussig, R. and M. Carlson, Nucl. Acids Res. 11:1943-1954 (1983)) under control of the yeast PGK promoter (Hitzeman, R.A., et al., Nucl. Acids Res. 10:7791-780 (1982)) as follows: The plasmid pM1D was digested with BsmI, treated with T4 DNA polymerase and then digested with XhoI and a restriction fragment containing V + $C_x$ was purified. This fragment was ligated to a similarly prepared restriction fragment from the plasmid, pING1149 which contains the PGK promoter (P) fused to the invertase signal sequence (S) to generate pR9D (Figure 5A). As the result of this fusion, the gene sequence encoding the mature form of the KM10 chimeric light chain was fused in frame to the gene sequence encoding the yeast invertase signal sequence (S). The PGK promoter-invertase signal sequence-chimeric light chain ($V, C_x$) fusion was cloned into a partial 2 micron circle ($2\mu$), ura3 yeast expression vector containing the PGK polyadenylation signal (Tm) to generate pX1D (Figure 5C).

The gene sequence encoding the mature form of the heavy chain variable region of KM10 and containing a BstEII site in the J region (as described in Example 1) and a Sst1 site introduced at the signal sequence processing site was fused to the human $C_H1$ region (which had been previously generated by introducing a stop codon in hinge, Robinson, R.R., et al., PCTU586/02269) in pING1453 to generate the KM10 Fd chain plasmid pF3D (see Figure 6).

The mature chimeric KM10 Fd gene from pF3D was next fused to the yeast invertase signal under the control of the yeast PGK promoter in a similar manner to that described for light chain generating pP12D (Figure 5B). The PGK promoter-invertase signal sequence-chimeric Fd chain ($V, C_H1$) fusion was cloned into a partial 2 micron circle ($2\mu$) expression vector containing the PGK polyadenylation signal (Tm) to generate pW6D (Figure 5D).

A single yeast expression vector containing both the chimeric light chain and Fd chain genes and their respective expression signals was constructed from pXID and pW6D. This final vector, pING3200, Figure 5E, contains a portion of 2 micron circle (oriY, REP3) and the two selectable markers leu2d and ura3.

### 4. Yeast Secretion of Chimeric KM10 Fab

The plasmid pING3200 was transformed into S. cerevisiae PS6 and the transformants were grown in broth under selective conditions as described above. The culture supernatants were assayed by ELISA and contained Fab levels of approximately 100 ng/ml.

The yeast strain that secreted 100 ng/ml Fab protein was grown in 50L of SD broth for 60 hr and Fab protein was purified from the culture supernatant.

### 5. Isolation of Chimeric Fab From Yeast and Production of Mouse Fab from KM10 Antibody

Fab was purified from 43L of culture supernatant. The culture supernatant was first concentrated by a DC10 unit over S10Y10 cartridge (Amicon), washing with 20L of distilled water, reconcentrating, and then washing with 10 mM sodium phosphate buffer at pH 8.0, and concentrating it again. The concentrate was then loaded onto a DE52 (Whatman) column pre-equilibrated with 10mM sodium phosphate buffer at pH 8.0. Sufficient 0.2M monosodium phosphate was added to the flow through of DE52 to adjust pH to 7.3, and the sample was concentrated over a YM10 membrane (Stirred Cell 2000, Amicon). The sample was then diluted with sufficient water and reconcentrated to 200 ml to give a conductivity of 1.6mS/cm. The total amount of protein was estimated by a colorimetric assay, and the sample was loaded onto a CM52 (Whatman) column at a ratio of 10mg total protein per g CM52 (pre-equilibrated with 10mM sodium phosphate buffer, pH 7.3). The CM52 column was eluted with sequential steps of 20 column volumes each of 2, 5, 10, 15, 20, 50, 100, 200, and 500mM NaCl in 10mM sodium phosphate buffer, pH 7.3. The fractions containing Fab as assessed by ELISA were combined and concentrated over a YM10 membrane to an Fab concentration of about 1 mg/ml, and stored frozen. The pooled fraction was further analyzed by SDS-PAGE and Western blotting. They both revealed a single 46 kD band consistent with the predicted molecular weight, based on nucleotide sequence.

## 6. Binding Characteristics of Fab Protein Secreted by Yeast.

The purification from yeast culture supernatants of protein of the expected size of Fab suggests that yeast secrete correctly folded, functional molecules. This was confirmed by performing direct and competition binding assays with the human carcinoma cell line LS174T. In the direct binding assay, Fab from yeast bound to the same target cancer cells as did mouse KM10 antibody, but not to a cell line which lacks the antigen. In the competition assay using $^{125}$I-labeled mouse KM10 antibody, the yeast-derived chimeric KM10 Fab inhibited binding of radio-labeled mouse KM10 antibody to human tumor cells (LS174T). Yeast-derived Fab caused a 50% inhibition of binding of mouse KM10 antibody at approximately 3.7 μg/ml (Table 3), similar to the inhibitory potency of KM10 mouse antibody. Yeast derived KM10 Fab inhibited binding of both intact mouse KM10 antibody and Fab fragments (prepared by papain digestion of mouse KM10 Fab prepared by papain digestion of mouse whole antibody.

Table 3.

| Inhibition of Binding of KM10 Antibody to HT-29 Tumor Cells | | | | |
| --- | --- | --- | --- | --- |
| | % Inhibition by Competing Antibody[a] | | | |
| Antibody Concentration μg/ml | Mouse KM10 | Chimeric KM10 Fab | Mouse KM10 Fab by papain | HumanIgG |
| 100 | 94 | 94 | 89 | 38 |
| 33.3 | 82 | 90 | 91 | 39 |
| 11.1 | 82 | 76 | 87 | 30 |
| 3.70 | 56 | 53 | 69 | 43 |
| 1.235 | 51 | 29 | 56 | 42 |
| 0.412 | 40 | 7 | 39 | 34 |
| 0.137 | 41 | 3 | | |

[a] - $^{125}$I-labeled KM10 antibody was incubated with LS174T tumor cells in the presence of the competing antibody at 4° C. Cells were washed free of unbound antibody, and cell-bound radioactivity was used to etermine the % inhibition of binding.

[b] - Human-IgG was used as a nonspecific antibody control.

## EXAMPLE 4

## A Chimeric Mouse-Human Fab with Human Tumor Cell Specificity Produced in E. coli

Bacteria are suited for production of chimeric antibodies expressed from mammalian cDNA since entire coding sequences can be expressed from well characterized promoters. E. coli is one of many useful bacterial species for production of foreign proteins (Holland et al., BioTechnology 4:427 (1986)) since a wealth of genetic information is available for optimization of its gene expression. E. coli can be used for production of foreign proteins internally or for secretion of proteins out of the cytoplasm, where they most often accumulate in the periplasmic space (Gray et al., Gene 39:247 (1985); Oka et al., Proc. Natl. Acad.

Sci. USA 82:7212 (1985)). Secretion from the E. coli cytoplasm has been observed for many proteins and requires a signal sequence. Proteins produced internally in bacteria are often not folded properly (Schoner et al., BioTechnology 3:151 (1985)). Protein secreted from bacteria, however, is often folded properly and assumes native secondary and tertiary structures (Hsiung et al., BioTechnology 4:991 (1986)).

An Fab molecule consists of two nonidentical protein chains linked by a single disulfide bridge. These two chains are the intact antibody light chain and the V, J, and $C_H1$ portions of the antibody heavy chain, Fd. The proper cDNA clones for the KM10 chimeric light and Fd genes have already been identified. In this example, these cDNA clones were organized into a single bacterial operon (a dicistronic message) as gene fusions to the pectate lyase (pelB) gene leader sequence from Erwinia carotovora (Lei et al., J. Bacteriol. 169:4379 (1987) and expressed from a strong regulated promoter. The result is a system for the simultaneous expression of two protein chains in E. coli. and the secretion of immunologically active, properly assembled Fab of KM10 chimeric antibody.

The following sections detail the secretion of chimeric KM10 Fab from E. coli.

## 1. Assembly of the pelB leader sequence cassette

Erwinia carotovora (EC) codes for several pectate lyases (polygalacturonic acid trans-eliminase) (Lei et al., Gene 35:63 (1985)). Three pectate lyase genes have been cloned, and the DNA sequence of these genes has been determined. When cloned into E. coli under the control of a strong promoter, the pelB gene is expressed and large quantities of pectate lyase accumulate in the periplasmic space and culture supernatant. The pelB signal sequence functions efficiently in E. coli and was used as a secretion signal for antibody genes in this example. (Other signal sequences would also be useful for this application.) The nucleotide sequence surrounding the signal sequence of the pelB gene is published (Lei et al., J. Bacteriol. 169:4379-4383 (1987)).

The pelB signal sequence contains a HaeIII restriction site at amino acid 22, adjacent to the signal peptidase cleavage site: ala-ala. Plasmid pSS1004 (Lei et al., J. Bacteriol. 169:4379-4383 (1987)) containing the pelB gene in pUC8 (Vierra and Messing, Gene 19:259 (1982)), was digested with HaeIII and EcoR1. This DNA was ligated with an eight base pair Sst1 linker to SspI and EcoR1 cut pBR322. The resulting plasmid contained a 300 bp fragment which included the 22 amino acid leader sequence of pelB and about 230 bp of upstream E. caratovora DNA. This plasmid, pING173, contains an insert that upon digestion with Sst1 and treatment with T4 DNA polymerase can be ligated directly to a DNA fragment flanked by the first amino acid of a mature coding sequence for any gene to generate a protein fusion containing a functional bacterial leader sequence in frame with the incoming gene. The Sst1 to EcoR1 restriction fragment in pING173 was cloned into pUC18 (Yanich-Perron et al., Gene 33:103 (1985)) to generate pRR175, which contains the pelB leader and adjacent upstream non-coding sequence (including a ribosome binding site) downstream of the lac promoter. Plasmid pING1500, derived from pRR175, contains only the region from the -48 of the pelB gene to an XhoI site downstream of the pelB leader, and includes the SstI site at the junction.

## 2. Preparation of Light Chain for Bacterial Expression

The intact KM10 chimeric light chain gene containing a BsmI restriction site at the signal sequence processing site and a unique XhoI site downstream of the gene in pM1D served as the starting point for bacterial expression. The plasmid pM1D was cut with BsmI, treated with T4 polymerase, and digested with XhoI. The approximately 800 bp fragment containing the light chain gene was purified and ligated to pING1500 that was cut with SstI, treated with T4 polymerase, and cut with XhoI (Figure 6A, B). The resulting plasmid that contained a pelB::KM10 light chain fusion was sequenced to determine that the proper in-frame fusion was formed. This plasmid was called pS2D.

## 3. Preparation of Fd for Bacterial Expression

The intact KM10 chimeric Fd gene containing a Sst1 restriction site at the signal sequence processing site and a XhoI restriction site downstream of the gene in pF3D served as the starting point for bacterial expression. The plasmid pF3D was cut with Sst1, treated with T4 polymerase, and digested with XhoI. The approximately 800 bp fragment containing the Fd gene was purified and ligated to pING1500 that was cut with SstI, treated with T4 polymerase, and cut with XhoI (Figure 6B, C). The resulting plasmid that contained a pelB::KM10 Fd fusion was sequenced to determine that the proper in-frame fusion was formed. This plasmid was called pQ16D.

### 4. Multicistronic expression system for light chain and Fd gene

For production of bacterially derived Fab, both light chain and Fd need to be produced simultaneously within the cell. Using the plasmids constructed with each of these genes separately, a series of expression vectors were constructed that contain both genes aligned so that transcription from a single promoter will specify both genes. This was done in a way that minimized the noncoding DNA between the two genes. Each gene has a ribosome binding site needed for translation initiation and the identical DNA sequence from -48 to the pelB leader::antibody gene junction. Plasmid PS2D was cut with SphI, treated with T4 polymerase, cut with EcoRI, and the vector fragment was purified (Figure 6D). Similarly, pQ16D was cut with XhoI, treated with T4 polymerase, cut with EcoRI and the fragment containing the Fd gene was purified (Figure 6E). These two purified DNA fragments were ligated to produce pB7E, which contained the two KM10 chimeric gene fusions linked in close proximity. The two gene cistron was placed under the control of the araB promoter in pING3104. Plasmid pB7E was cut with SphI, treated with T4 polymerase, cut with XhoI, and the fragment containing the Fd and x genes was purified (Figure 6G). This DNA fragment was ligated to the vector fragment from pING3104 that had been cut with EcoRI, treated with T4 polymerase, and cut with XhoI (Figure 6F), generating pING3202. This vector contains all the necessary features for expression of KM10 chimeric Fab in E. coli.

### 5. Production of Chimeric KM10 Fab in Bacteria

Expression of KM10 chimeric Fab from pING3202 in E. coli is under the inducible control of the araB promoter. Upon arabinose induction, Fab secreted into the growth medium increased more than 10 fold. Uninduced bacterial colonies harboring pING3202 were phenotypically indistinguishable from E. coli harboring pING3104. The strain harboring pING3202 was cultured in 10L of minimal medium, supplemented with 0.7% glycerol as the carbon source, and induced with 0.2% arabinose for over 12 hr. Fab was detected in the fermentation broth by ELISA. The Fab can be purified from this fermentation broth and has properties identical to those of the chimeric Fab described above. KM10 Fab produced in bacterial binds to LS174T cells.

## CONCLUSIONS

The examples presented above demonstrate a number of important characteristics of the chimeric KM10 antibody and the genetically engineered KM10 Fab proteins of the invention. Firstly, both the chimeric KM10 antibody and its Fab derivative bind to human tumor cell lines to the same extent as the mouse KM10 antibody with approximately the same avidity. The chimeric KM10 antibody is significant because it binds to the surface of human tumor cells. The KM10 mAb has minimal reactivity to normal cells such as fibroblasts, endothelial cells, or epithelial cells in the major organs. Thus, the chimeric KM10 mAb defines an antigen that is useful in vitro for distinguishing human tumor cells from non-tumor cells, and thus has utility as diagnostic agent for in vitro use.

Although the prospect of attempting tumor therapy using mAb is attractive, to date such mAb therapy has been met with only limited success (Houghton, et al., Proc. Natl. Acad. Sci. 82:1242-1246 (Feb. 1985)). The therapeutic efficacy of unmodified mouse mAb appears to be too low for most practical purposes. Chimeric KM10 antibody is an improved therapeutic agent over mouse KM10 mAb for the treatment of human tumors in vivo. First, the high biological activity of the chimeric KM10 antibody against human tumor cell lines combined with minimal reactivity with normal tissues indicates that this antibody can mediate selective destruction of malignant tissue. Second, the "more human" chimeric KM10 antibody is more resistant to rapid clearance from the body than the mouse KM10 antibody. Third, this enhanced presence in the circulation (and presumably in the tissues) means that the chimeric KM10 antibodies and their derivatives can be advantageously used for in vivo diagnosis and therapy of tumors in the form of an immunoconjugate with drugs, toxins, immunomodulators, radionuclides, etc. Such immunoconjugates, and techniques to form them, are known to those skilled in the art and can be used to modify the chimeric KM10 antibody within the scope of the present invention.

Deposits

Two illustrative cell lines secreting chimeric KM10 antibody were deposited on May 5, 1989, prior to the U.S. filing date, at the ATCC, Rockville Maryland. These are:

1. Transfected hybridoma Sp2/0 (pING2240 and pING2242), Strain C739, designated as ATCC #HB 10131; and

2. Saccharomyces cerevisiae PS6 (pING3200), strain G267, designated as ATCC #20945

**Claims**

1. A polynucleotide molecule comprising a cDNA sequence coding for the variable region of an immunoglobulin chain having specificity to the antigen bound by the murine KM10 monoclonal antibody.

2. The molecule of claim 1 wherein said chain is a heavy chain.

3. The molecule of claim 1 wherein said chain is a light chain.

4. The molecule of claim 1 which further comprises an additional sequence coding for the constant region of a human immunoglobulin chain, both said sequences in operable linkage with each other.

5. The molecule of claim 4 wherein said additional sequence is a cDNA sequence.

6. The molecule of claim 1 which is a recombinant DNA molecule.

7. The molecule of claim 6 which is in double stranded DNA form.

8. The molecule of claim 7 which is an expressible vehicle.

9. The molecule of claim 8 wherein said vehicle is a plasmid.

10. A prokaryotic host transformed with the molecule of claim 4.

11. The host of claim 10 which is a bacterium.

12. A eukaryotic host transfected with the molecule of claim 4.

13. The host of claim 12 which is a yeast cell or a mammalian cell.

14. An immunoglobulin heavy chain comprising a constant human region and a variable region having specificity to the antigen bound by the KM10 murine monoclonal antibody.

15. An immunoglobulin light chain comprising a constant human region and a variable region having specificity to the antigen bound by the KM10 murine monoclonal antibody.

16. A chimeric antibody molecule comprising two light chains and two heavy chains, each of said chains comprising a constant human region and a variable region having specificity to the antigen bound by the KM10 murine monoclonal antibody.

17. The antibody of claim 16 in detectably labelled form.

18. The antibody of claim 16 immobilized on an aqueous-insoluble solid phase.

19. A process of preparing an immunoglobulin heavy chain having a constant human region and a variable region having specificity to the antigen bound by the KM10 murine monoclonal antibody which comprises:
culturing a host capable of expressing said chain under culturing conditions; and recovering from said culture said heavy chain.

20. A process of preparing an immunoglobulin light chain having a constant human region and a' variable region with specificity to the antigen bound by the KM10 murine monoclonal antibody which comprises:
culturing a host capable of expressing said chain under culturing conditions; and recovering from said culture said light chain.

21. A process of preparing a chimeric immunoglobulin containing a heavy chain and a light chain, each of said heavy and light chains having a constant human region and a variable region with specificity to the antigen bound by the KM10 murine monoclonal antibody which comprises:
culturing a host capable of expressing said heavy chain, or said light chain, or both, under culturing conditions; and
recovering from said culture said chimeric immunoglobulin molecule.

22. The process of any of claims 19, 20 or 21 wherein said host is prokaryotic.

23. The process of any of claims 19, 20 or 21 wherein said host is eukaryotic.

19

EP 0 404 003 A2

24. An immunoassay method for the detection of an antigen capable of binding to the KM10 murine monoclonal antibody, in a sample which comprises:
contacting said sample with the antibody of claim 16; and
detecting whether said antibody binds to said antigen.

25. An in vivo or in vitro imaging method to detect an antigen capable of binding to the KM10 murine monoclonal antibody which comprises contacting said antigen with the labelled antibody of claim 17 and detecting said antibody.

26. A method of killing cells carrying an antigen thereon, which antigen is capable of binding to the KM10 murine monoclonal antibody, which comprises:
contacting said cells with the antibody of claim 16 and allowing said killing to occur.

27. The method of claim 26 wherein said killing occurs by complement-mediated lysis of said cells.

28. The method of claim 26 wherein said killing occurs by ADCC.


Claims for the following Contracting State: ES

1. A process for preparing a polynucleotide molecule comprising a cDNA sequence encoding the variable region of an immunoglobulin chain having specificity to the antigen bound by the murine KM10 monoclonal antibody which comprises
    (a) cultivating the mouse B cell hybridoma line producing the monoclonal antibody;
    (b) separating a fraction containing the monoclonal antibody messenger RNA; and
    (c) preparing a full-length cDNA library from purified messenger RNA from which the appropriate variable region gene fragments of the chain genes can be identified with appropriate probes, sequenced and made compatible with a constant gene segment.

2. The process according to claim 1, wherein said chain is a light chain and wherein said chain genes are genes encoding the light chains.

3. The process according to claim 1, wherein said chain is a heavy chain and wherein said chain genes are genes encoding the heavy chains.

4. The process according to claim 1, wherein the polynucleotide molecule further comprises an additional sequence coding for the constant region of a human immunoglobulin chain both said sequences in operable linkages with each other.

5. The process according to claim 4, wherein said additional sequence is a cDNA sequence.

6. The process according to claim 1, wherein the molecule is a recombinant DNA molecule.

7. The process according to claim 6, wherein the molecule is in double stranded DNA form.

8. The process according to claim 7, wherein the molecule is an expressible vehicle.

9. The process according to claim 8, wherein said vehicle is a plasmid.

10. A process of transforming a prokaryotic host which comprises transferring the molecule of claim 4 into appro priate expression vehicles and introducing the recombinant vector into the prokaryotic host to transform it.

11. The process of claim 10, wherein the host is a bacterium.

12. A process for transforming an eukaryotic host which comprises transferring the molecule of claim 7 into appropriate expression vehicles for expression and introducing the recombinant vector into the host to transform it.

13. The process of claim 12, wherein the host is a yeast cell or a mammalian cell.

14. A process of preparing an immunoglobulin heavy chain having a constant human region and a variable region having specificity to the antigen bound by the KM10 murine monoclonal antibody which comprises:
cultering a host capable of expressing said chain under culturing conditions; and recovering from said culture said heavy chain.

15. A process of preparing an immunoglobulin light chain having a constant human region and a variable region with specificity to the antigen bound by the KM10 murine monoclonal antibody which comprises:
culturing a host capable of expressing said chain under culturing conditions; and recovering from said culture said light chain.

16. A process of preparing a chimeric immunoglobulin containing a heavy chain and a light chain, each of said heavy and light chains having a constant human region and a variable region with specificity to the antigen bound by the KM10 murine monoclonal antibody which comprises:
culturing a host capable of expressing said heavy chain, or said light chain, or both, under culturing conditions; and

20

recovering from said culture said chimeric immunoglobulin molecule.

17. The process according to claim 16, wherein the antibody is in detectable labelled form.

18. The process according to claim 16, wherein the antibody is immobilized on an aqueous insoluble solid phase.

19. The process of any of claims 14, 15 or 16, wherein said host is prokaryotic.

20. The process of any of claims 14, 15 or 16, wherein said host is eukaryotic.

21. An immunoassay method for the detection of an antigen capable of binding to the KM10 murine monoclonal antibody, in a sample which comprises:

contacting said sample with the antibody as prepared according to claim 16; and detecting whether said antibody binds to said antigen.

22. An in vivo or in vitro imaging method to detect an antigen capable of binding to the KM10 murine monoclonal antibody which comprises contacting said antigen with the labelled antibody prepared according to claim 17 and detecting said antibody.

23. A method of killing cells carrying an antigen thereon, which antigen is capable of binding to the KM10 murine monoclonal antibody, which comprises:

contacting said cells with the antibody prepared according to claim 16 and allowing said killing to occur.

24. The method of claim 23, wherein said killing occurs by completement-mediated lysis of said cells.

25. The method of claim 23, wherein said killing occurs by ADCC.

EP 0 404 003 A2

KM10 Heavy Chain DNA Sequence

```
                                                                SalI
                                                   3'-CGGGTTCCAGCAGCTG
ATACCAGCAAAGGGAGTGACCAGTTTGTCTTAAGGCACCACTGAGCCCAAGGTCTTAGAC

TAGTACCTA-5'                                       3'-GTTTCACGGGCTCGA
   METAspTrpLeuTrpLysLeuLeuPheLeuMETAlaAlaAlaGlnSerAlaGlnAla
ATCATGGATTGGCTGTGGAAGTTGCTATTCCTGATGGCAGCTGCCCAAAGTGCCCAAGCA
                                                            ↓
                                                          SstI

GTCTAGGTCAAC-5'
GlnIleGlnLeuValGlnSerGlyProGluLeuMETLysProGlyGluThrValLysIle
CAGATCCAGTTGGTGCAGTCTGGACCTGAACTCATGAAGCCTGGAGAGACAGTCAAGATC

SerCysLysAlaSerGlyTyrThrPheThrAsnTyrGlyMETAsnTrpValLysGlnAla
TCCTGCAAGGCTTCTGGTTATACCTTCACAAACTATGGAATGAACTGGGTGAAGCAGGCT

ProGlyLysGlyLeuLysTrpMETGlyTrpIleAsnThrTyrThrGlyGluProThrTyr
CCAGGAAAGGGTTTAAAGTGGATGGGCTGGATAAACACCTACACTGGAGAGCCAACATAT

AlaAspAspPheLysGlyArgPheAlaPheSerLeuGluThrSerValSerThrGlyHis
GCTGATGACTTCAAGGGACGGTTTGCCTTCTCTCTGGAGACCTCTGTCAGCACTGGCCAT

LeuGlnIleAsnAsnLeuLysAsnGluAspThrAlaThrTyrPheCysAlaArgTrpGly
TTGCAGATCAACAACCTCAAAAATGAGGACACGGCTACATATTTCTGTGCAAGATGGGGG
                                                    BstEII
                                      3'-CCTTGGAGCCAGTGGCAGAG-5'
GlySerTyrGlyMETAspTyrTrpGlyGlnGlyThrSerValThrValSerSerAlaLys
GGTTCCTATGGTATGGACTACTGGGGTCAAGGAACCTCAGTCACCGTCTCCTCAGCCAAA
```

# FIG. 1

KM10 Light Chain DNA Sequence

```
                              SalI
              3'-GTGATTAATCGGCAGCTGGTTTTAGG-5'      METAspPhe
CGGGGGATAAGACTAGCACTAATTAGCCAGAGACCAAAATCCAAATACACAATGGACTTT
         15              30          45              60


                              3'-CAGTGTCACAGCTTACGTCTTTAACAC
ArgValGlnIlePheSerPheLeuLeuIleSerValThrValSerArgGlyGluIleVal
CGGGTGCAGATTTTCAGCTTCCTGCTAATCAGTGTCACAGTGTCCAGAGGAGAAATTGTG
         75              90             ↓             120
                                              BsmI
GAG-5'
LeuThrGlnSerProValIleAlaAlaAlaSerLeuGlyGlnLysValThrIleThrCys
CTCACTCAGTCTCCAGTCATCGCAGCTGCATCTCTGGGGCAAAAGGTCACCATCACCTGC
         135             150             165             180


SerAlaSerSerSerValSerTyrMETTyrTrpTyrGlnGlnLysSerGlyThrSerPro
AGTGCCAGCTCAAGTGTAAGTTACATGTACTGGTACCAACAGAAGTCAGGCACCTCCCCC
         195             210             225             240


LysProTrpIleTyrGlyIleSerLysLeuAlaSerGlyValProThrArgPheSerGly
AAACCATGGATTTATGGAATATCCAAACTGGCTTCTGGAGTCCCAACTCGCTTCAGTGGC
         255             270             285             300


SerGlySerGlyThrSerTyrSerLeuThrIleSerSerValGluAlaGluAspAlaAla
AGTGGGTCTGGGACCTCTTACTCTCTCACAATCAGCAGCGTGGAGGCTGAAGATGCTGCC
         315             330             345             360
                                                    HindIII
                                           3'-CCCTGGTTCGAA
IleTyrTyrCysGlnGlnTrpAsnTyrProLeuIleThrPheGlyAlaGlyThrLysLeu
ATTTATTACTGCCAGCAGTGGAATTATCCTCTTATCACGTTCGGTGCTGGGACCAAGCTG
         375             390             405             420

CTCGAC-5'
GluLeuLys
GAGCTGAAA
         435
```

FIG.2

FIG.3A

FIG. 3B

*FIG.4A*

FIG. 4B

EP 0 404 003 A2

*FIG.5*

EP 0 404 003 A2

FIG.6A

FIG. 6B